# EUROPEAN PATENT APPLICATION

(11) **EP 4 621 071 A1**
(43) Date of publication of application: **24.09.2025**
(21) Application number: 24382315.0
(22) Date of filing: 22.03.2024
(51) Int. Cl.: C12Q 1/6883

(54) **IN VITRO METHOD FOR PREDICTING THE RESPONSE OF PATIENTS SUFFERING FROM RHEUMATOID ARTHRITIS TO A TREATMENT WITH TUMOR NECROSIS FACTOR (TNF) INHIBITORS**

(71) Applicant: Fundación Pública Galega Instituto de Investigación Sanitaria de Santiago de Compostela, 15706 Santiago de Compostela A Coruña (ES)
(72) Inventor: DÍAZ PEÑA, Roberto, 15706 Santiago de Compostela (ES); CASTRO SANTOS, Patricia, 15706 Santiago de Compostela (ES); SANTIAGO LAMELAS, Lucía, 15706 Santiago de Compostela (ES)
(74) Representative: Hoffmann Eitle

(57) **Abstract**

The present invention refers to a method for the identification of biomarkers or biomarker signatures for predicting the response of patients suffering from rheumatoid arthritis to a treatment with tumor necrosis factor (TNF) inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

## Description

### FIELD OF THE INVENTION

The present invention refers to the medical field. Particularly, the present invention refers to a method for the identification of biomarkers or biomarker signatures for predicting the response of patients suffering from rheumatoid arthritis to a treatment with tumor necrosis factor (TNF) inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

### STATE OF THE ART

Rheumatoid arthritis (RA) is a chronic, systemic, inflammatory disease affecting approximately 0.5-1% of the global population, having more prevalence in females than un males. Is characterized by inflammation of the synovial joints, which leads to progressive joint destruction, disability, and reduced quality of life. In particular, 80% of insufficient treated patients develops misaligned joints and a half of them are unable to work within ten years after the diagnosis. Treatment strategy at the diagnosis begins with lifestyle counselling and non-steroid anti-inflammatory drugs (NSAIDs). Next step are the glucocorticoids and since nineties the denominated DMARDs (disease modifying anti-rheumatic drugs), in which stands out the tumour necrosis factor (TNF)-α, the TNF inhibitors and the TNF-receptor inhibitors. Anti-TNF agents have revolutionized the treatment of RA, significantly improved clinical outcomes and inhibited radiographic progression in a substantial proportion of patients. However, although anti-TNF therapy is effective in most cases, up to 30% of treated patients show an inadequate response. Despite all the recent knowledge about the RA, there are some challenges that need to be solved. Specifically, due to the heterogeneity of the disease and because of the elevated risk of unfavourable health problems, such as infections, there is an urge to predict the therapy response.

Therefore, there is an unmet medical need of finding reliable tool, particularly biomarkers, aimed at predicting the response of RA patients to TNF inhibitors treatment. The present invention is focused on solving this problem, thus providing a reliable method for the identification of biomarkers/biomarker signatures for predicting the response to TNF inhibitors in RA patients, for classifying RA patients into responders or non-responders to this treatment and, finally, for monitoring whether RA patients are responding to the therapy in order to decide or recommend whether TNF inhibitors treatment should be used.

### DESCRIPTION OF THE INVENTION

### Brief description of the invention

The present invention refers to an *in vitro* method for the identification of biomarkers or biomarker signatures for predicting the response of patients suffering from rheumatoid arthritis to a treatment with TNF inhibitors. Moreover, the present invention refers to the use of the identified biomarkers or biomarker signatures for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

Particularly, the inventors of the present invention herein show that transcriptomic data could serve as a prognostic and predictive biomarker in RA patients under anti-TNF therapy. So, a bioinformatic analysis was performed in which a transcriptomic signature was reported, as proof of concept, with high capacity to predict the response to anti-TNF treatment in patients with RA, prior to its initiation.

Although, a explained above, a specific transcriptomic signature comprising the genes KCNK17, GLS2, GTPBP2, DNTTIP1, IL18R1 and COMTD1 was reported as proof of concept (see **Example 2**), the following tables (**Table 1, Table 2** and **Table 3**) show several signatures comprising at least two genes selected from the list consisting of: KCNK17, GLS2, GTPBP2, DNTTIP1, IL18R1, COMTD1, FEM1C, MRPL24 and/or MYPOP, or combinations thereof comprising between 2 and 9 of these genes, as reliable signatures for performing the method of the invention in different data sets, all of them showing AUC ≥ 0.8.

**Table 1**

| **Gene combination** | **AUC** |
|---|---|
| KCNK17+GLS2+DNTTIP1+IL18R1 | 0.816135084 |
| KCNK17+GLS2+DNTTIP1+FEM1C | 0.804252658 |
| KCNK17+DNTTIP1+IL18R1+FEM1C | 0.813633521 |
| GLS2+GTPBP2+DNTTIP1+FEM1C | 0.801751094 |
| GLS2+DNTTIP1+IL18R1+FEM1C | 0.814258912 |
| KCNK17+GLS2+GTPBP2+DNTTIP1+IL18R1 | 0.818636648 |
| KCNK17+GLS2+GTPBP2+DNTTIP1+FEM1C | 0.818011257 |
| KCNK17+GLS2+GTPBP2+IL18R1+FEM1C | 0.811131957 |
| KCNK17+GLS2+DNTTIP1+IL18R1+COMTD1 | 0.816135084 |
| KCNK17+GLS2+DNTTIP1+IL18R1+FEM1C | 0.828017511 |
| KCNK17+GLS2+DNTTIP1+COMTD1+FEM1C | 0.804252658 |
| KCNK17+GLS2+IL18R1+COMTD1+FEM1C | 0.802376485 |
| KCNK17+GTPBP2+DNTTIP1+IL18R1+FEM1C | 0.811131957 |
| KCNK17+DNTTIP1+IL18R1+COMTD1+FEM1C | 0.814258912 |
| GLS2+GTPBP2+DNTTIP1+IL18R1+FEM1C | 0.817385866 |
| GLS2+GTPBP2+DNTTIP1+COMTD1+FEM1C | 0.801125704 |
| GLS2+DNTTIP1+IL18R1+COMTD1+FEM1C | 0.816135084 |
| **KCNK17+GLS2+GTPBP2+DNTTIP1+IL18R1+COMTD1** | 0.816760475 |
| KCNK17+GLS2+GTPBP2+DNTTIP1+IL18R1+FEM1C | 0.837398374 |
| KCNK17+GLS2+GTPBP2+DNTTIP1+COMTD1+FEM1C | 0.815509694 |
| KCNK17+GLS2+GTPBP2+IL18R1+COMTD1+FEM1C | 0.820512821 |
| KCNK17+GLS2+DNTTIP1+IL18R1+COMTD1+FEM1C | 0.83427142 |
| KCNK17+GTPBP2+DNTTIP1+IL18R1+COMTD1+FEM1C | 0.813633521 |
| GLS2+GTPBP2+DNTTIP1+IL18R1+COMTD1+FEM1C | 0.824265166 |

| | |
|---|---|
| *Dataset GSE138746 (39 R vs. 41 NR)* | |

**Table 2**

| **Gene combination** | **AUC** |
|---|---|
| KCNK17+GLS2+GTPBP2+DNTTIP1 | 0.82010582 |
| GLS2+GTPBP2+DNTTIP1+FEM1C | 0.80952381 |
| KCNK17+GLS2+GTPBP2+DNTTIP1+COMTD1 | 0.804232804 |
| KCNK17+GLS2+GTPBP2+DNTTIP1+FEM1C | 0.814814815 |
| GLS2+MYPOP+IL18R1+COMTD1+FEM1C | 0.804232804 |
| **KCNK17+GLS2+GTPBP2+DNTTIP1+IL18R1+COMTD1** | 0.835978836 |
| KCNK17+GLS2+GTPBP2+DNTTIP1+IL18R1+FEM1C | 0.804232804 |
| KCNK17+MRPL24+MYPOP+DNTTIP1+IL18R1+COMTD1 | 0.80952381 |
| GLS2+MRPL24+MYPOP+GTPBP2+IL18R1+COMTD1 | 0.825396825 |
| GLS2+MRPL24+MYPOP+IL18R1+COMTD1+FEM1C | 0.80952381 |
| GLS2+MYPOP+GTPBP2+IL18R1+COMTD1+FEM1C | 0.80952381 |

| | |
|---|---|
| *Dataset GSE224842 (21 R vs. 9 NR)* | |

**Table 3**

| **Gene combination** | AUC |
|---|---|
| MRPL24+GTPBP2 | 0.800925926 |
| GTPBP2+IL18R1 | 0.800925926 |
| KCNK17+MRPL24+GTPBP2 | 0.800925926 |
| KCNK17+GTPBP2+IL18R1 | 0.803240741 |
| MRPL24+GTPBP2+DNTTIP1 | 0.800925926 |
| MRPL24+GTPBP2+IL18R1 | 0.805555556 |
| GTPBP2+DNTTIP1+IL18R1 | 0.805555556 |
| GTPBP2+IL18R1+COMTD1 | 0.821759259 |
| GTPBP2+COMTD1+FEM1C | 0.805555556 |
| KCNK17+MRPL24+GTPBP2+DNTTIP1 | 0.800925926 |
| KCNK17+MRPL24+GTPBP2+IL18R1 | 0.819444444 |
| KCNK17+GTPBP2+DNTTIP1+IL18R1 | 0.800925926 |
| KCNK17+GTPBP2+IL18R1+COMTD1 | 0.821759259 |
| KCNK17+GTPBP2+IL18R1+FEM1C | 0.800925926 |
| KCNK17+GTPBP2+COMTD1+FEM1C | 0.803240741 |
| GLS2+MRPL24+GTPBP2+IL18R1 | 0.800925926 |
| GLS2+MRPL24+GTPBP2+FEM1C | 0.800925926 |
| GLS2+GTPBP2+IL18R1+COMTD1 | 0.828703704 |
| GLS2+GTPBP2+IL18R1+FEM1C | 0.803240741 |
| GLS2+GTPBP2+COMTD1+FEM1C | 0.805555556 |
| MRPL24+GTPBP2+DNTTIP1+IL18R1 | 0.805555556 |
| MRPL24+GTPBP2+IL18R1+COMTD1 | 0.81712963 |
| MRPL24+GTPBP2+IL18R1+FEM1C | 0.81712963 |
| GTPBP2+DNTTIP1+IL18R1+COMTD1 | 0.80787037 |
| GTPBP2+IL18R1+COMTD1+FEM1C | 0.814814815 |
| KCNK17+GLS2+MRPL24+GTPBP2+IL18R1 | 0.819444444 |
| KCNK17+GLS2+GTPBP2+IL18R1+COMTD1 | 0.821759259 |
| KCNK17+GLS2+GTPBP2+COMTD1+FEM1C | 0.805555556 |
| KCNK17+MRPL24+GTPBP2+DNTTIP1+IL18R1 | 0.824074074 |
| KCNK17+MRPL24+GTPBP2+DNTTIP1+FEM1C | 0.803240741 |
| KCNK17+MRPL24+GTPBP2+IL18R1+COMTD1 | 0.831018519 |
| KCNK17+MRPL24+GTPBP2+IL18R1+FEM1C | 0.824074074 |
| KCNK17+MRPL24+GTPBP2+COMTD1+FEM1C | 0.800925926 |
| KCNK17+GTPBP2+DNTTIP1+IL18R1+COMTD1 | 0.80787037 |
| KCNK17+GTPBP2+DNTTIP1+IL18R1+FEM1C | 0.80787037 |
| KCNK17+GTPBP2+IL18R1+COMTD1+FEM1C | 0.819444444 |
| GLS2+MRPL24+GTPBP2+DNTTIP1+IL18R1 | 0.803240741 |
| GLS2+MRPL24+GTPBP2+IL18R1+COMTD1 | 0.814814815 |
| GLS2+MRPL24+GTPBP2+IL18R1+FEM1C | 0.819444444 |
| GLS2+MRPL24+GTPBP2+COMTD1+FEM1 C | 0.800925926 |
| GLS2+GTPBP2+DNTTIP1+IL18R1+COMTD1 | 0.810185185 |
| GLS2+GTPBP2+DNTTIP1+IL18R1+FEM1C | 0.80787037 |
| GLS2+GTPBP2+IL18R1+COMTD1+FEM1C | 0.828703704 |
| MRPL24+GTPBP2+DNTTIP1+IL18R1+COMTD1 | 0.821759259 |
| MRPL24+GTPBP2+DNTTIP1+IL18R1+FEM1C | 0.803240741 |
| MRPL24+GTPBP2+DNTTIP1+COMTD1+FEM1C | 0.803240741 |
| MRPL24+GTPBP2+IL18R1+COMTD1+FEM1C | 0.8125 |
| GTPBP2+DNTTIP1+IL18R1+COMTD1+FEM1C | 0.810185185 |
| KCNK17+GLS2+MRPL24+GTPBP2+DNTTIP1+IL18R1 | 0.824074074 |
| KCNK17+GLS2+MRPL24+GTPBP2+IL18R1+COMTD1 | 0.833333333 |
| KCNK17+GLS2+MRPL24+GTPBP2+IL18R1+FEM1C | 0.824074074 |
| KCNK17+GLS2+MRPL24+GTPBP2+COMTD1+FEM1C | 0.80787037 |
| **KCNK17+GLS2+GTPBP2+DNTTIP1+IL18R1+COMTD1** | 0.81712963 |
| KCNK17+GLS2+GTPBP2+DNTTIP1+IL18R1+FEM1C | 0.800925926 |
| KCNK17+GLS2+GTPBP2+IL18R1+COMTD1+FEM1C | 0.821759259 |
| KCNK17+MRPL24+GTPBP2+DNTTIP1+IL18R1+COMTD1 | 0.828703704 |
| KCNK17+MRPL24+GTPBP2+DNTTIP1+IL18R1+FEM1C | 0.824074074 |
| KCNK17+MRPL24+GTPBP2+DNTTIP1+COMTD1+FEM1C | 0.803240741 |
| KCNK17+MRPL24+GTPBP2+IL18R1+COMTD1+FEM1C | 0.810185185 |
| KCNK17+GTPBP2+DNTTIP1+IL18R1+COMTD1+FEM1C | 0.8125 |
| GLS2+MRPL24+GTPBP2+DNTTIP1+IL18R1+COMTD1 | 0.824074074 |
| GLS2+MRPL24+GTPBP2+DNTTIP1+IL18R1+FEM1C | 0.805555556 |
| GLS2+MRPL24+GTPBP2+IL18R1+COMTD1+FEM1C | 0.81712963 |
| GLS2+GTPBP2+DNTTIP1+IL18R1+COMTD1+FEM1C | 0.810185185 |
| MRPL24+GTPBP2+DNTTIP1+IL18R1+COMTD1+FEM1C | 0.821759259 |

| | |
|---|---|
| *Dataset GSE33377 (18 R vs. 24 NR)* | |

So, the first embodiment of the present invention refers to an *in vitro* method for the identification of biomarkers or biomarker signatures for predicting the response of patients suffering from rheumatoid arthritis to a treatment with tumor necrosis factor (TNF) inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors which comprises: a) Assessing the level of expression of at least a gene selected from the list consisting of: KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis who are responder patients to treatment with TNF inhibitors, b) assessing the level of expression of at least a gene selected from the list consisting of KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis who are non-responder patients to treatment with TNF inhibitors, and c) wherein the identification of a deviation of the level of expression of at least a gene selected from the list consisting of KCNK17, GLS2, GTPBP2, DNTTIP1, IL18R1, COMTD1, FEM1C, MRPL24 and/or MYPOP, or any combination thereof, across responder and non-responder subjects, is an indication that the gene/s can be used as biomarkers or biomarker signatures for predicting the response of patients suffering from rheumatoid arthritis to treatment with TNF inhibitors, or for classifying patients suffering from rheumatoid arthritis into responder or non-responder to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

The second embodiment of the present invention refers to an *in vitro* method for predicting the response of patients suffering from rheumatoid arthritis to a treatment with TNF inhibitors, or for classifying patients suffering from rheumatoid arthritis into responder or non-responder patients to a treatment with TNF inhibitors, for monitoring patients suffering from rheumatoid arthritis to assess whether they are responding to treatment with TNF inhibitors, or for deciding or recommending whether to treat patients suffering from rheumatoid arthritis with TNF inhibitors, which comprises: a) Assessing the level of expression of at least a gene selected from the list consisting of: KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis, b) wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors, or that the patient is responding to treatment with TNF inhibitors, or c) wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

In a preferred embodiment the methods described above comprise: a) Assessing the level of expression of a combination of genes selected from **Table 1, Table 2** or **Table 3,** in a biological sample obtained from patients suffering from rheumatoid arthritis, b) wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors, or that the patient is responding to treatment with TNF inhibitors, or c) wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

The third embodiment of the present invention refers to the *in vitro* use of at least a gene selected from the list consisting of: KCNK17, GLS2, GTPBP2, DNTTIP1, IL18R1, COMTD1, FEM1C, MRPL24 and/or MYPOP, or of a kit comprising reagents for the identification of the level of expression of at least a gene selected from the list consisting of: KCNK17, GLS2, GTPBP2, DNTTIP1, IL18R1, COMTD1, FEM1C, MRPL24 and/or MYPOP, or any combination thereof, for predicting the response of patients suffering from rheumatoid arthritis to a treatment with TNF inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

In a preferred embodiment the present invention refers to the *in vitro* use of a combination of genes selected from **Table 1, Table 2** or **Table 3,** for predicting the response of patients suffering from rheumatoid arthritis to a treatment with TNF inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

The fourth embodiment of the present invention refers to TNF inhibitors for use in a method of treatment of rheumatoid arthritis, wherein the method comprises assessing whether the patient would be a responder patient to a treatment with TNF inhibitors by assessing the level of expression of at least a gene selected from the list consisting of: KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis, wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors; or wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors. Alternatively, the present invention refers to a method for treating a patient suffering from rheumatoid arthritis with TNF inhibitors, wherein the method comprises a first step of determining whether the patient would be a responder patient to a treatment with TNF inhibitors by assessing the level of expression of at least a gene selected from the list consisting of: KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis, wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors; or wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

In a preferred embodiment the present invention refers to TNF inhibitors for use in a method of treatment of rheumatoid arthritis, wherein the method compromises assessing whether the patient would be a responder patient to a treatment with TNF inhibitors by assessing the level of expression of a combination of genes selected from **Table 1, Table 2** or **Table 3,** in a biological sample obtained from patients suffering from rheumatoid arthritis, wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors, or that the patient is responding to treatment with TNF inhibitors; or wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors. Alternatively, the present invention refers to a method for treating a patient suffering from rheumatoid arthritis with TNF inhibitors, wherein the method comprises a first step of determining whether the patient would be a responder patient to a treatment with TNF inhibitors by assessing whether the patient would be a responder patient to a treatment with TNF inhibitors by assessing the level of expression of a combination of genes selected from **Table 1, Table 2** or **Table 3,** in a biological sample obtained from patients suffering from rheumatoid arthritis, wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors, or that the patient is responding to treatment with TNF inhibitors; or wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

In a preferred embodiment, the biological sample is blood, serum or plasma.

In a preferred embodiment, the TNF inhibitors is selected from: infliximab, etanercept, golimumab, certolizumab and adalimumab.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of". Thus, the phrase "consisting of" indicates that the listed elements are required or mandatory, and that no other elements may be present.
- According to the present invention, a reference value can be a "pre-established threshold value" or a "cut-off" value. Typically, a "threshold value" or "cut-off value" can be determined experimentally, empirically, or theoretically. According to the present invention, the "pre-established threshold" value refers to a value previously determined in patients suffering for rheumatoid arthritis who are non-responders to a treatment with TNF inhibitors. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### Description of the figures

**Figure 1****.** Flowchart utilized to search of a transcriptomic signature that predicts response to anti-TNF treatment.
**Figure 2****.** Differential expression analysis of the GSE138746 dataset.
**Figure 3****.** Predictive model consisting of 6 genes was selected to effectively discriminate R (responders) and NR (non-responders), where the results showed high area under the curve (AUC) values using three different datasets.
**Figure 4****.** Receiver operating characteristic curves of each gene using the GSE224842 dataset.
**Figure 5****.** Receiver operating characteristic curves of each gene using the GSE33377 dataset.

### Detailed description of the invention

The present invention is illustrated by means of the Examples set below without the intention of limiting its scope of protection.

### Example 1. Material and methods

### Example 1.1. Data source

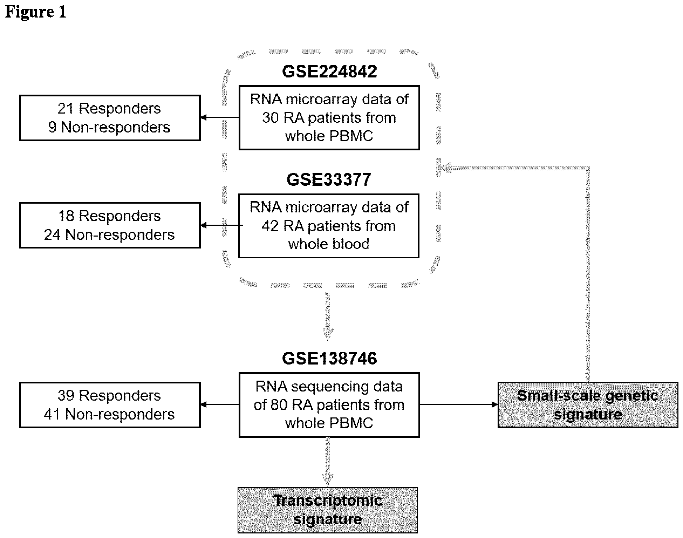

The study scheme for uncovering and testing the capacity of a transcriptomic signature to predict anti-TNF response is shown in **Figure 1****.** We performed a retrospective analysis of whole peripheral blood mononuclear cells (PBMC) transcriptomic profiling for patients with RA, to define the small-scale signature genes involved in the response to anti-TNF treatment. The Gene Expression Omnibus (GEO) database (https://www.ncbi.nlm.nih.gov/geo/) numbered GSE138746 was obtained [Tao W, Concepcion AN, Vianen M, Marijnissen ACA, Lafeber FPGJ, Radstake TRDJ, et al. Multiomics and Machine Learning Accurately Predict Clinical Response to Adalimumab and Etanercept Therapy in Patients With Rheumatoid Arthritis. Arthritis Rheumatol. 2021;73(2):212-22]*.* This dataset contained 80 samples with gene expression profiling on PBMC, including 38 and 42 samples from RA patients treated with adalimumab and etanercept, respectively. Two more publicly available datasets (GSE224842 and GSE33377) profiled by microarray was downloaded from GEO database to define, based on the small-scale signature genes, a transcriptomic signature capable of predicting response prior to anti-TNF treatment. GSE224842 dataset contained expression profiling on PBMC for 30 RA patients initiating treatment with abatacept, whereas the GSE33377 dataset contained expression profiling on whole blood for 42 RA patients starting treatment with infliximab or adalimumab [Toonen EJM, Gilissen C, Franke B, Kievit W, Eijsbouts AM, den Broeder AA, et al. Validation study of existing gene expression signatures for anti-TNF treatment in patients with rheumatoid arthritis. PLoS One. 2012; 7(3):e33199].

### Example 1.2. Differentially expressed genes analysis

To perform an initial analysis of differential expression, the statistical analyses were performed using R version 4.1.1. After processing the expression matrix of the GSE138746 dataset using the DESeqDataSetFromMatrix R function, principal component study and differential expression analysis were performed with the DESeq function *[*Love MI, Huber W, Anders S. Moderated estimation of fold change and dispersion for RNA-seq data with DESeq2. Genome Biol. 2014;15(12):550]*.* Differential gene expression analysis was done using the parametric Wald test with the Benjamini-Hochberg adjustment method. Those genes with counts mean lower than 5 were removed from the DESeq table before the p-value adjustment. The GSEA algorithm was used for enrichment analysis of Wikipathways and GO terms (cellular component, biological processes and molecular function) *[*Yu G, Wang LG, Han Y, He QY. clusterProfiler: an R package for comparing biological themes among gene clusters. OMICS. 2012;16(5):284-7]*.*

### Example 1.3. Genetic small-scale signature

The identification of a transcriptomic signature capable of predicting response prior to anti-TNF treatment constitutes a phenotype prediction problem. This category of problems is characterized by a high degree of under-determinacy, given that the number of monitored genes far exceeds the number of samples employed. It is imperative to prioritize genes based on their discriminatory power in predicting the phenotype and to focus on sampling highly predictive networks anticipated to be involved in genetic pathways underlying treatment response.

Mathematically, these networks inhabit the uncertainty space associated with the corresponding classifier used for phenotype discrimination. The mathematical structure of uncertainty spaces in the context of linear and nonlinear inverse problems has been previously elucidated [Fernández Martinez JL, Fernández Muñiz MZ, Tompkins MJ. On the topography of the cost functional in linear and nonlinear inverse problems. GEOPHYSICS. 2012;77(1):W1-15*][*Fernández-Martinez JL, Fernández-Muñiz Z, Pallero JLG, Pedruelo-González LM. From Bayes to Tarantola: New insights to understand uncertainty in inverse problems. Journal of Applied Geophysics. 2013;98:62-72]*.* Within a given classifier, the smallest-scale signature is defined as one comprising the fewest discriminatory genes with the highest predictive accuracy. However, the presence of noise in genetic data implies that some highly discriminatory signatures may not directly correspond to relevant genetic pathways *[*deAndrés-Galiana EJ, Fernández-Martinez JL, Sonis ST. Sensitivity analysis of gene ranking methods in phenotype prediction. J Biomed Inform. 2016;64:255-64][Deandres-Galiana EJ, Fernández-Martinez JL, Saligan LN, Sonis ST. Impact of Microarray Preprocessing Techniques in Unraveling Biological Pathways. J Comput Biol. 2016;23(12):957-68]*.* In addressing parameter identification problems, the significance of noise necessitates its integration into the evaluation of identified solutions, underscoring the importance of appraising the discriminatory power of key genes related to the phenotype using sampling methodologies.

In this research, the Fisher's Ratio Sampler has been applied *[*Cernea A, Fernández-Martinez JL, deAndrés-Galiana EJ, Fernández-Ovies FJ, Fernández-Muñiz Z, Alvarez-Machancoses O, et al. Sampling Defective Pathways in Phenotype Prediction Problems via the Fisher's Ratio Sampler. En: Rojas I, Ortuño F, editores. Bioinformatics and Biomedical Engineering. Cham: Springer International Publishing; 2018. p. 15-23], involving the sampling of defective pathways based on the discriminatory power of individual genes as measured by the Fisher's ratio. This entails identifying genes with the highest Fisher's ratio within the set of genes exhibiting the highest fold change. Specifically, genes that are differentially expressed in both tails (over and under-expressed) are identified, and these genes are ranked based on their Fisher's ratio, which seeks genes that effectively separate the classes and exhibit low intra-class variance. The set of discriminatory genes is defined as those that are differentially expressed and possess a Fisher's ratio greater than 0.8. The Fisher's ratio cutoff value is a tunable parameter in this procedure, which can be adjusted, if necessary, to a minimum value of fr = 0.5 when the number of discriminatory genes is limited within this set.

To establish a small-scale genetic signature, the most discriminatory genes are ranked in decreasing order according to their discriminatory power. The algorithm subsequently determines the small-scale signature that optimally discriminates between classes through recursive feature elimination. Predictive accuracy estimation is conducted through Leave-One-Out-Cross-Validation (LOOCV) using a nearest neighbour classifier. The small-scale signature provides an approximate representation of the typical length (number of genes) of high discriminatory networks.

The random sampler is employed to identify alternative networks of highly discriminatory genes, utilizing a prior sampling probability for any individual gene proportional to its Fisher's ratio. Following the random construction of a network based on the Fisher's probability distribution, its LOOCV predictive accuracy is established. This sampling process adheres to Bayes' rule, incorporating a prior probability contingent on the Fisher's ratio of the selected genes and a likelihood probability function dependent on the LOOCV predictive accuracy of the network.

Ultimately, considering the most discriminatory networks with a predictive accuracy exceeding a predefined threshold (typically greater than 85%), subsequent sampling frequencies of the primary prognostic genes involved in these networks are determined.

### Example 2. Results

### Example 2.1. Analysis of differential expression genes of the GSE138746 dataset

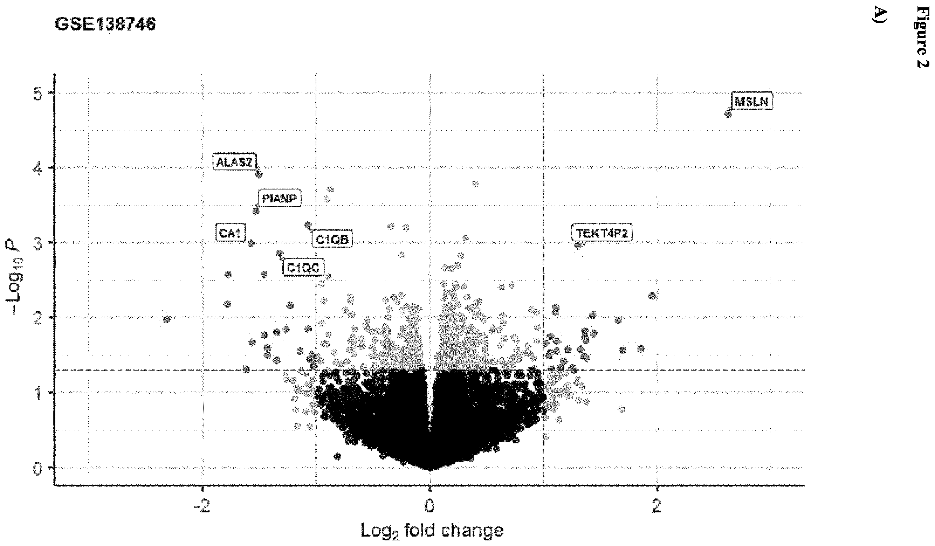

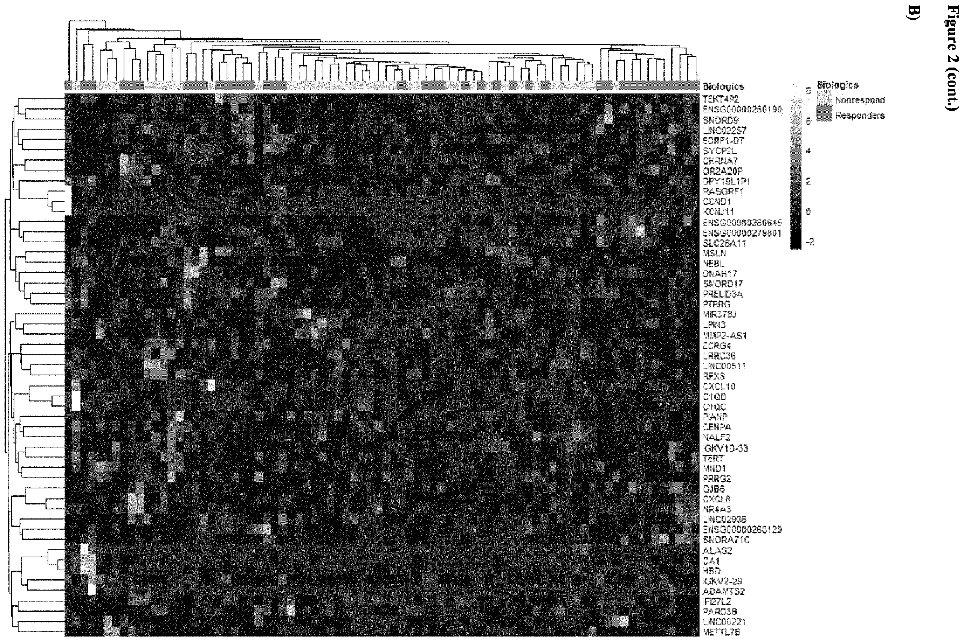

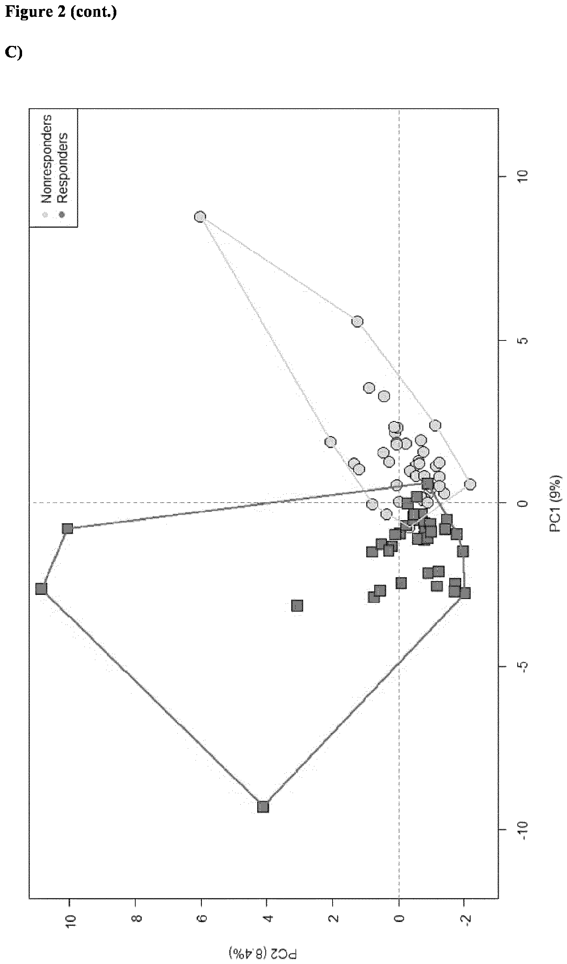

Based on an initial analysis of the RNA-seq results, a total of 53 differentially expressed genes were identified between responders (N) and non-responders (NR), including 28 up-regulated genes and 25 down-regulated genes in R *versus* NR, according to the criteria: Fold Change > |1| and *p*-value <0.05. To visualize the differential expression genes, we constructed a volcano-plot **(****Figure 2A****).** No significant differences were found between both groups when the p-value was adjusted by the Benjamini-Hochberg adjustment method. In fact, the representation of the heatmap and the Principal Component Analysis (PCA) analysis show a poor separation of the samples from responders (yellow) and non-responders (red) to anti-TNF therapy according the 53 differentially expressed genes found by RNA-seq analysis (**Figure 2B** and **Figure 2C**, respectively). Functional enrichment analysis does not report any significant relation between the analyzed genes and the response (all the pathways show p-values>0.05).

### Example 2.2. A transcriptomic signature was associated with the prediction of response to anti-TNF therapy

**Table 1** shows the list of most discriminatory genes of the phenotypes of the responder patients compared with no-responders (most discriminatory genes using the Fisher's Ratio Sampler).

**Table 1**

| Gene | MedianC1 | StdC1 | MedianC2 | StdC2 | FC | FR | LOOCV |
|---|---|---|---|---|---|---|---|
| KCNK17 | 1.0 | 13.28 | 15.0 | 13.04 | -0.92 | 0.57 | 67.50 |
| LOC100506235 | 29.0 | 15.90 | 13.0 | 14.49 | 0.64 | 0.55 | 68.75 |
| TRBV6-4 | 12.0 | 12.45 | 0.0 | 11.71 | 0.93 | 0.49 | 70.00 |
| GLS2 | 68.0 | 37.01 | 35.0 | 30.20 | 0.53 | 0.48 | 68.75 |
| MRPL24 | 456.0 | 107.41 | 356.0 | 110.06 | 0.20 | 0.42 | 72.50 |
| DNTTIP1 | 639.0 | 197.64 | 464.0 | 183.20 | 0.27 | 0.42 | 72.50 |
| MYPOP | 101.0 | 51.91 | 150.0 | 56.29 | -0.33 | 0.41 | 78.75 |
| LPIN3 | 12.0 | 16.64 | 0.0 | 9.49 | 1.22 | 0.39 | 73.75 |
| OR2A9P | 16.0 | 12.90 | 4.0 | 15.38 | 0.20 | 0.36 | 75.00 |
| GTPBP2 | 1824.0 | 341.08 | 1539.0 | 337.47 | 0.12 | 0.35 | 70.00 |
| ZFTA | 141.0 | 56.53 | 189.0 | 58.65 | -0.20 | 0.35 | 76.25 |
| COMTD1 | 199.0 | 65.24 | 150.0 | 51.84 | 0.37 | 0.35 | 77.50 |
| ZFP57 | 7.0 | 67.25 | 72.0 | 89.25 | -0.59 | 0.34 | 81.25 |
| IL18R1 | 377.0 | 136.53 | 508.0 | 181.10 | -0.36 | 0.33 | 86.25 |
| KRBOX5 | 324.0 | 81.78 | 398.0 | 99.33 | -0.20 | 0.33 | 86.25 |
| FEM1C | 597.0 | 166.85 | 764.0 | 242.94 | -0.20 | 0.32 | 86.25 |
| ENSG00000278627 | 4.0 | 9.79 | 13.0 | 12.51 | -0.78 | 0.32 | 86.25 |
| THBS4-AS1 | 48.0 | 27.61 | 27.0 | 25.11 | 0.52 | 0.32 | 88.75 |
| ECRG4 | 16.0 | 15.97 | 5.0 | 11.49 | 1.02 | 0.31 | 86.25 |
| ENSG00000215447 | 120.0 | 48.23 | 84.0 | 43.71 | 0.32 | 0.31 | 82.50 |
| LRRC75A | 98.0 | 39.62 | 68.0 | 37.10 | 0.28 | 0.31 | 78.75 |
| NDUFA8 | 340.0 | 102.98 | 262.0 | 97.07 | 0.22 | 0.30 | 83.75 |
| RPS24P19 | 15.0 | 6.14 | 10.0 | 6.68 | 0.31 | 0.30 | 82.50 |
| COPG2 | 526.0 | 150.26 | 419.0 | 124.08 | 0.29 | 0.30 | 81.25 |

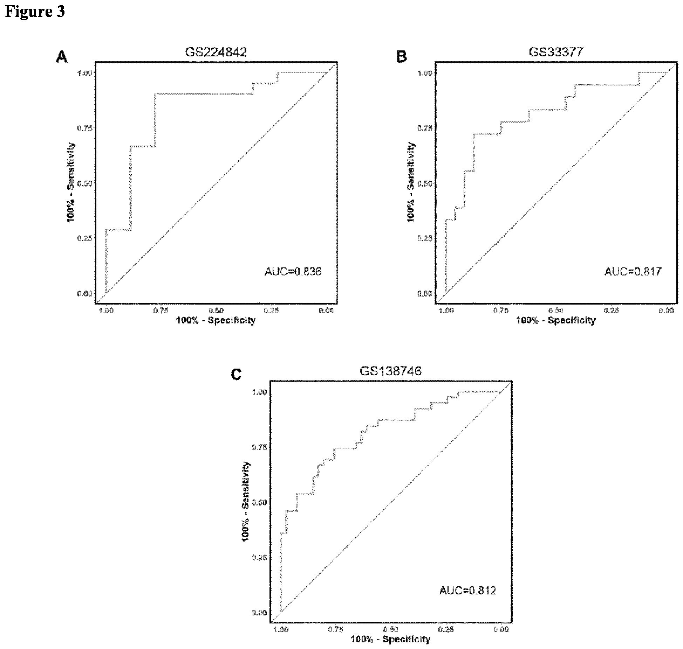

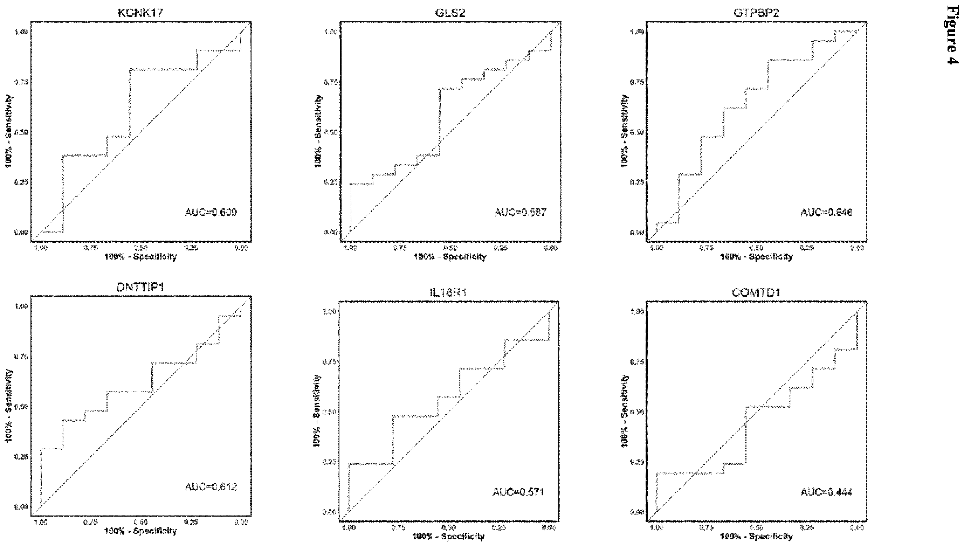

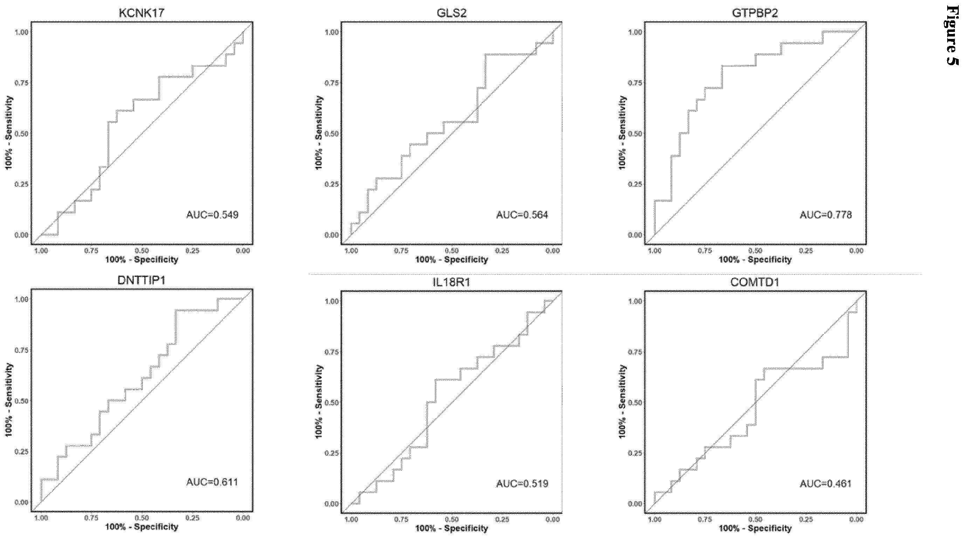

The small-scale genetic signature found was composed of the 18 most discriminatory genes with an LOOCV predictive accuracy of 88.75%. Next, we evaluated the discriminatory power of these genes to create a transcriptomic signature that could accurately distinguish between these patient groups with a minimal number of genes using ROC analyses. Thus, a predictive model composed of 6 genes was selected to effectively discriminate R and NR, where the results showed high area under the curve (AUC) values using the GSE224842 and GSE33377 datasets. **Figure 3A** and **Figure 3B****,** respectively. Single genes showed a low to moderate discriminatory power to distinguish between responders and non-responders (AUC= 0.44-0.78, **Figure 4** and **Figure 5**). Then, we performed an internal validation using first dataset (GSE138746). ROC analyses were conducted to combine the 6 genes of the model, which showed a high AUC (AUC= 0.81, **Figure 3C**).

## Claims

1. *In vitro* method for the identification of biomarkers or biomarker signatures for predicting the response of patients suffering from rheumatoid arthritis to a treatment with tumor necrosis factor (TNF) inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors which comprises:
a. Assessing the level of expression of at least a gene selected from the list consisting of: KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis who are responder patients to treatment with TNF inhibitors,
b. Assessing the level of expression of at least a gene selected from the list consisting of KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis who are non-responder patients to treatment with TNF inhibitors,
c. Wherein the identification of a deviation of the level of expression of at least a gene selected from the list consisting of KCNK17, GLS2, GTPBP2, DNTTIP1, IL18R1, COMTD1, FEM1C, MRPL24 and/or MYPOP, or any combination thereof, across responder and non-responder subjects, is an indication that the gene/s can be used as biomarkers or biomarker signatures for predicting the response of patients suffering from rheumatoid arthritis to treatment with TNF inhibitors, or for classifying patients suffering from rheumatoid arthritis into responder or non-responder to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

2. *In vitro* method for predicting the response of patients suffering from rheumatoid arthritis to a treatment with TNF inhibitors, or for classifying patients suffering from rheumatoid arthritis into responder or non-responder patients to a treatment with TNF inhibitors, for monitoring patients suffering from rheumatoid arthritis to assess whether they are responding to treatment with TNF inhibitors, or for deciding or recommending whether to treat patients suffering from rheumatoid arthritis with TNF inhibitors, which comprises:
a. Assessing the level of expression of at least a gene selected from the list consisting of: KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis,
b. Wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors, or that the patient is responding to treatment with TNF inhibitors, and/or
c. Wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

3. *In vitro* method, according to claim 2, which comprises:
a. Assessing the level of expression of a combination of genes selected from **Table 1, Table 2** or **Table 3,** in a biological sample obtained from patients suffering from rheumatoid arthritis,
b. Wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors, or that the patient is responding to treatment with TNF inhibitors, and/or
c. Wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

4. *In vitro* use of at least a gene selected from the list consisting of: KCNK17, GLS2, GTPBP2, DNTTIP1, IL18R1, COMTD1, FEM1C, MRPL24 and/or MYPOP, or of a kit comprising reagents for the identification of the level of expression of at least a gene selected from the list consisting of: KCNK17, GLS2, GTPBP2, DNTTIP1, IL18R1, COMTD1, FEM1C, MRPL24 and/or MYPOP, or any combination thereof, for predicting the response of patients suffering from rheumatoid arthritis to a treatment with TNF inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

5. *In vitro* use, according to claim 4, of a combination of genes selected from **Table 1, Table 2** or **Table 3,** for predicting the response of patients suffering from rheumatoid arthritis to a treatment with TNF inhibitors, or for classifying patients suffering from rheumatoid arthritis into responder or non-responder patients to a treatment with TNF inhibitors, or for monitoring patients suffering from rheumatoid arthritis to assess whether they are responding to a treatment with TNF inhibitors, or for deciding or recommending whether to treat patients suffering from rheumatoid arthritis with TNF inhibitors.

6. TNF inhibitor for use in a method of treatment of rheumatoid arthritis, wherein the method comprises assessing whether the patient would be a responder patient to a treatment with TNF inhibitors by assessing the level of expression of at least a gene selected from the list consisting of: KCNK17, GLS2, GTPBP2, DNTTIP1, IL18R1, COMTD1, FEM1C, MRPL24 and/or MYPOP, or any combination thereof, in a biological sample obtained from patients suffering from rheumatoid arthritis, wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors; and/or wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

7. TNF inhibitor for use, according to claim 8, wherein the method compromises assessing whether the patient would be a responder patient to a treatment with TNF inhibitors by assessing the level of expression of a combination of genes selected from **Table 1, Table 2** or **Table 3,** in a biological sample obtained from patients suffering from rheumatoid arthritis, wherein the identification of a higher expression level of the genes: KCNK17, IL18R1, FEM1C and/or MYPOP as compared with a pre-established threshold value measured in non-responder patients, is an indication that the patient will respond to treatment with TNF inhibitors, or that the patient is responding to treatment with TNF inhibitors; and/or wherein the identification of a higher expression level of the genes: GLS2, GTPBP2, DNTTIP1, COMTD1 and/or MRPL24 as compared with a pre-established threshold value measured in non-responder patients, is an indication that the patient will not respond to treatment with TNF inhibitors, or that the patient is not responding to treatment with TNF inhibitors.

8. *In vitro* method, according to any of the claims 1 to 3, *in vitro* use, according to any of the claims 4 or 5, or TNF inhibitor for use according to any of the claims 6 or 7, wherein the biological sample is blood, serum or plasma.

9. *In vitro* method, according to any of the claims 1 to 3, *in vitro* use, according to any of the claims 4 or 5, or TNF inhibitor for use according to any of the claims 6 or 7, wherein the TNF inhibitors is selected from: infliximab, etanercept, golimumab, certolizumab and adalimumab.
